(19) Europäisches Patentamt
European Patent Office
Office européen des brevets

(11) EP 1 762 608 A1

(12) **EUROPEAN PATENT APPLICATION**

(43) Date of publication:
**14.03.2007 Bulletin 2007/11**

(51) Int Cl.:
*C12M 3/00* (2006.01)   *B01L 3/00* (2006.01)

(21) Application number: **06251023.5**

(22) Date of filing: **27.02.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **09.09.2005 JP 2005262832**

(71) Applicant: **FUJITSU LIMITED**
**Kawasaki-shi,**
**Kanagawa 211-8588 (JP)**

(72) Inventors:
• **Yabuki, Akihiko,**
**c/o Fujitsu Limited**
**Kawasaki-shi**
**Kanagawa 211-8588 (JP)**
• **Sakai, Satoru,**
**c/o Fujitsu Limited**
**Kawasaki-shi**
**Kanagawa 211-8588 (JP)**

(74) Representative: **Mohun, Stephen John**
**HASELTINE LAKE**
**Imperial House**
**15-19 Kingsway**
**London WC2B 6UD (GB)**

(54) **Petri dish for trapping cell**

(57)     A petri dish that includes a cell trapping portion having a plurality of suction holes. A cell-contained liquid is placed in the petri dish and the cell-contained liquid is sucked from the suction holes, which are smaller that the cells, from below the petri dish to trap the cells in the suction holes. The petri dish includes a liquid retaining portion having a space of a capacity that allows sucked-liquid, which is liquid sucked through the suction holes and that do not contain cells, to be retained therein, and configured so that an interface between the sucked-liquid in the space and gas being positioned at a lower level than a surface level of the cell-contained liquid in the petri dish.

FIG.1

**Description**

BACKGROUND OF THE INVENTION

1. Field of the Invention

The present invention relates to a petri dish used for trapping biological cells in suction holes by sucking a liquid that contains cells through the suction holes.

2. Description of the Related Art

Recently, in the field of life science, specifically in the fields of regenerative medicine and genome-based drug discovery, by cultivating a cell and injecting a gene or a drug into the cell, the property of the cell is modified. To carry out such works, a means for efficiently setting cells in place is required.

Therefore, the technology as follows is developed in the following manner. That is, a portion which is a wide cross-sectional area of a flow path is provided in the middle of the flow path for flowing a liquid containing cells, a flow rate of the liquid is made slow to trap cells in the portion, and a wall surface in the portion having the wide cross-sectional area is chemically modified to increase an adhesion force of adhering cells thereto (see, for example, Japanese Patent Application Laid-Open No. 2004-180555, Japanese Patent Application Laid-Open No. 2004-163).

However, even if the chemical modification is to be subjected to the wall surface of the cell trapping portion, the adhesion force becomes insufficient by the time when a hole is made in a cell using a needle to inject a gene or a drug into the cell, and hence, the cell may move.

To resolve these problems, the technology as follows is developed. A cell trapping plate is prepared in which a plurality of suction holes smaller than external dimensions (about 10 micrometers to 100 micrometers) of a cell are formed. Cells are trapped in the suction holes by sucking the cells using a suction pump from the underside of the cell trapping plate through the suction holes, to inject a gene or a drug into the cell by a microinjection method (see, for example, specification of Japanese Patent Application No. 2005-14588, specification of Japanese Patent Application No. 2004-284756, and specification of Japanese Patent Application No. 2005-102094).

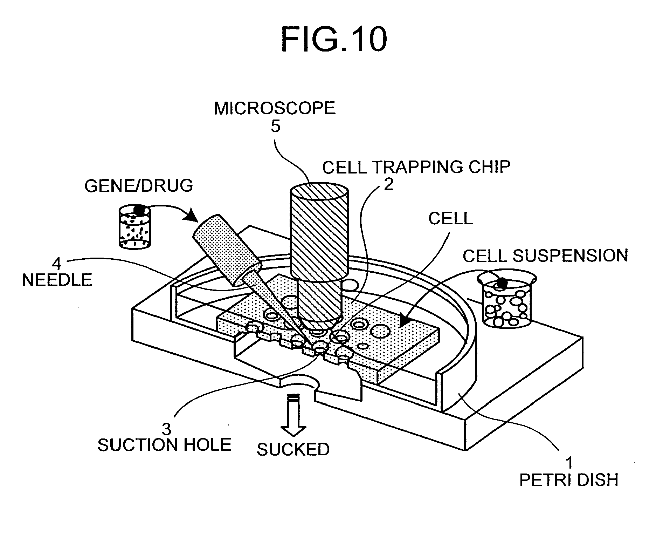

Fig. 10 is a diagram for explaining a conventional microinjection method. As shown in Fig. 10, in the microinjection method, a cell trapping chip 2 made from silicone being a cell trapping plate is provided in a petri dish 1 for containing a cell suspension, and a liquid is sucked using a suction pump from the underside of the cell trapping chip 2, to trap cells in suction holes 3. It is noted that PBS (Phosphate Buffer Saline) or the like is used as a liquid for suspending cells therein.

A needle 4 of an extra fine capillary tube is filled with a gene/drug, and the needle 4 is stuck to a cell to inject the gene/drug into the cell. The introducing work of the gene/drug toward the cell using the microinjection method is carried out under a microscope 5.

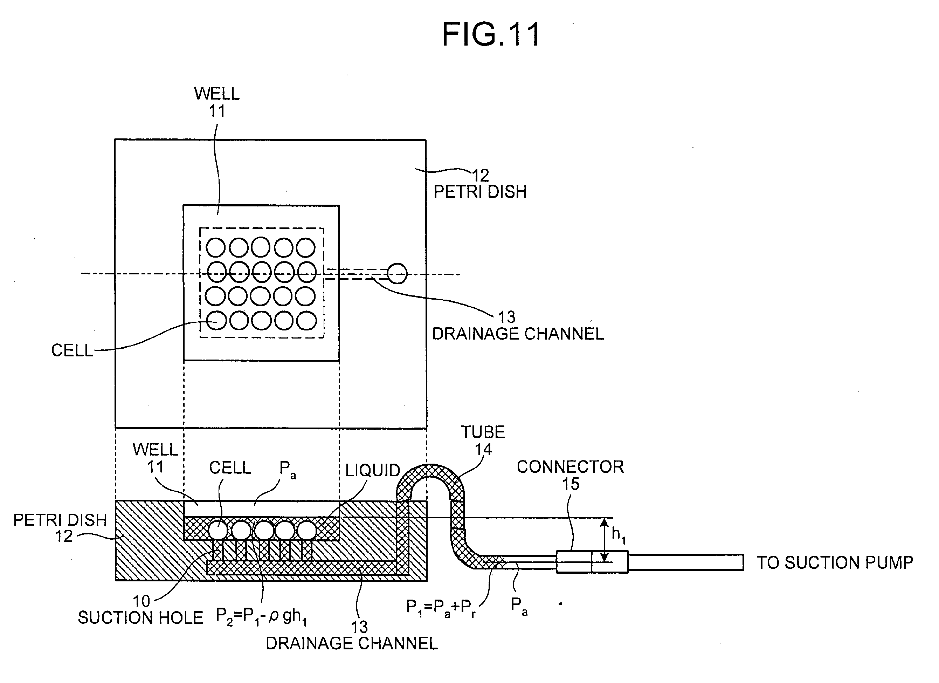

Fig. 11 is a diagram for explaining a pressure state when a cell is trapped using a conventional petri dish. The petri dish has a drainage channel 13. Liquid is discharged to the outside of a petri dish 12 from the drainage channel 13 when the liquid in a well 11 is sucked through suction holes 10 for trapping cells. The drainage channel 13 is connected to a tube 14, which can flexibly bend, in the top surface of the petri dish 12, and the tube 14 is connected to a suction pump (not shown) via a connector 15.

When such a petri dish 12 is used, before cells are supplied, the liquid is sucked by a suction force with which the surface boundary of the liquid formed in the suction holes 10 can be broken, and the suction holes 10 and the drainage channel 13 are filled with the liquid. By supplying cells while continuing the suction, the cells can be trapped in the suction holes 10, so that a gene or a drug can be injected into each cell.

However, there still remains such a problem that when a petri dish has to be carried after a gene or a drug is injected into each cell, it is difficult to continue trapping cells if suction of the liquid is stopped. For example, when the petri dish is to be carried from one place to another, the tube 14 needs to be removed. But, if the tube 14 is moved in its vertical direction, the height of the liquid level in the tube 14 changes, this may cause the cells to be released from the suction holes 10.

More specifically, as shown in Fig. 11, the following relation among those as follows holds, where $P_a$ is atmospheric pressure, $P_1$ is pressure of the liquid in the tube 14 at a location lower from the liquid level of the well 11 by $h_1$, $P_2$ is pressure of the liquid in the liquid level of the well 11, $\rho$ is liquid density, g is gravity acceleration, and $P_r$ is Laplace pressure of the liquid in the tube 14.

$$P_1 = P_a + P_r,$$

$$P_2=P_1-\rho gh_1=Pa-\rho gh1+Pr$$

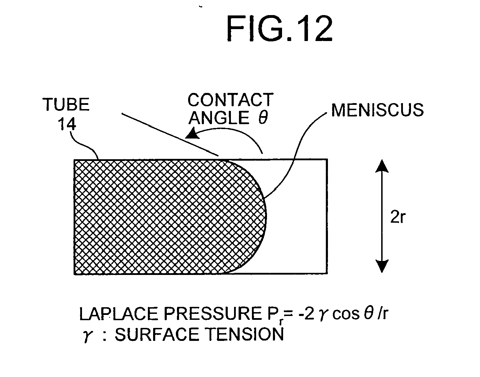

Here, as shown in Fig. 12, the Laplace pressure $P_r$ is expressed by the following equation, where $\theta$ is a contact angle of a meniscus portion, 2r is an internal diameter of the tube 14, and $\gamma$ is a surface tension of the liquid.

$$P_r=-2\gamma\cos\theta/r$$

Furthermore, referring to a balance of pressure on the liquid level of the well 11, the following relation holds for

$$P_a-P_2=0$$

and the following relation is obtained from these equations

$$h_1=P_r/\rho g$$

To trap cells in the suction holes 10, a difference between heights of the liquid level in the well 11 and of the liquid in the tube 14 needs to be set to $h_1$ or higher. However, in the conventional petri dish 12, when the tube 14 is moved in its vertical direction and the difference between the heights becomes $h_1$ or less, the cells are released from the suction holes 10.

This is because trapping pressure for trapping a cell becomes $\rho g\Delta h$ when the difference between the heights changes from $h_1$ by $\Delta h$, but the trapping pressure becomes negative because $\Delta h<0$ when the difference between the heights becomes $h_1$ or less.

Thus, in the conventional petri dish 12, when the tube 14 is removed from the petri dish 12, the liquid leaks from the tube 14, and this causes the liquid having leaked to contaminate peripheral environment.

Further, conventionally, because extra cells are removed after the cells are trapped in the suction holes, the PBS is continuously supplied into the petri dish where the cells trapped and the extra cells coexist, and discharged therefrom.

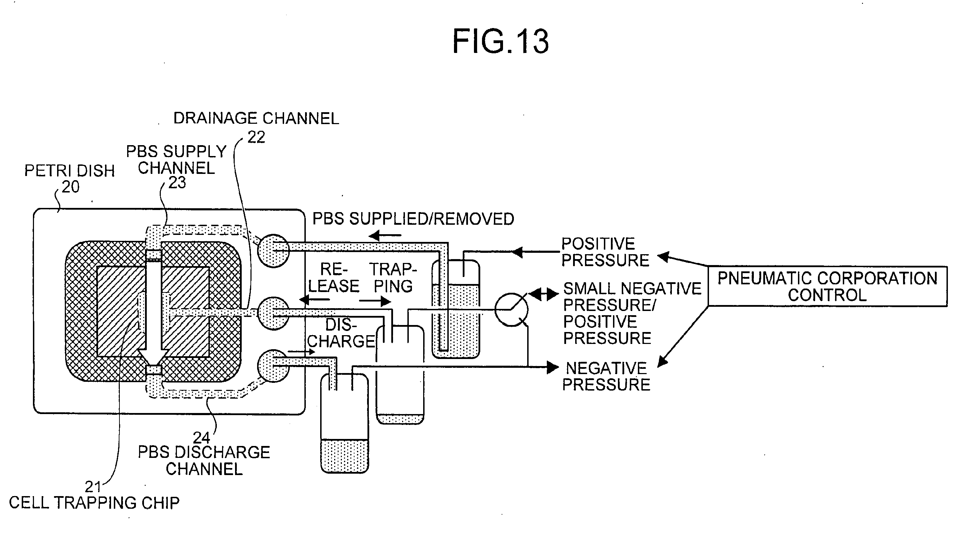

Fig. 13 is a diagram for explaining a conventional PBS supply-discharge system. The PBS supply-discharge system has a cell trapping chip 21 provided in a petri dish 20, and further includes a drainage channel 22 for discharging a liquid from the petri dish 20 via the suction holes of the cell trapping chip 21, a PBS supply channel 23 for supplying PBS into the petri dish 20, and a PBS discharge channel 24 for discharging PBS from the petri dish 20.

The supply and discharge of PBS are performed by pneumatic control. However, if the supply amount of PBS is smaller than its discharge amount, the surface of the cell trapping chip 21, where cells are trapped, dries. Therefore, it is necessary to balance the supply of PBS with the discharge thereof, but it is difficult to realize it by the pneumatic control.

Conventionally, there is known a petri dish that has an angular slope portion between a first well including a cell trapping chip 30 and a second well for retaining extra cells which are not trapped in the cell trapping chip, and that the extra cells in the first well are forcedly flowed over the slope portion by the PBS to be retained in the second well.

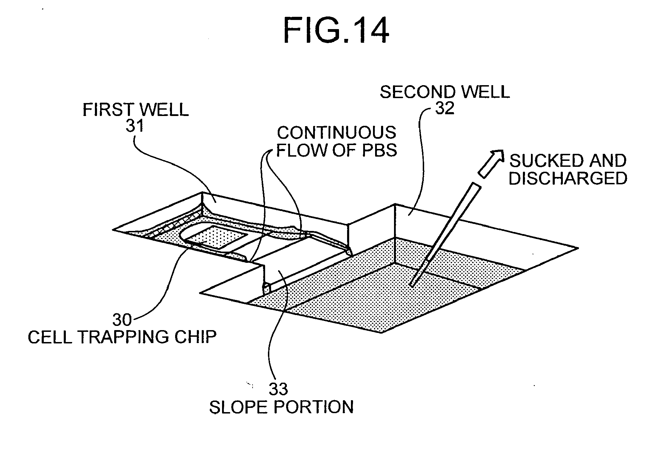

Fig. 14 is a diagram for explaining a slope portion 33 of a first well 31 in which the cell trapping chip 30 is provided. As shown in Fig. 14, in the petri dish, the slope portion 33 is formed at a fluid outlet of the first well 31 from which the liquid is discharged to a second well 32.

As shown in Fig. 14, however, it is difficult to shut off a continuous flow of the PBS, simply by providing the angular slope portion, produced along the wall sides of the first well 31 by the influence of surface tension. Therefore, the liquid in the first well 31 flows to the second well 32, to cause the surface of the cell trapping chip 30 to dry.

Thus, there is a need to develop an easy-to-use microinjection device. In other words, there is a need to consider how to maintain the state where the cells are trapped in the suction holes even during the carriage of the petri dish, how to prevent leakage of the liquid when the petri dish is removed from the microinjection device, and how to prevent the cell trapping chip from drying when the extra cells are flowed away.

## SUMMARY OF THE INVENTION

It is an object of the present invention to at least solve the problems in the conventional technology.

According to an aspect of the present invention, a petri dish that includes a cell trapping portion having a plurality of suction holes, a cell-contained liquid containing biological cells is placed in the petri dish and the cell-contained liquid is sucked from the suction holes, which are smaller that the cells, from below the petri dish to trap the cells in the suction holes, includes a liquid retaining portion having a space of a capacity that allows sucked-liquid, which is liquid sucked through the suction holes and that do not contain cells, to be retained therein, and configured so that an interface between the sucked-liquid in the space and gas being positioned at a lower level than a surface level of the cell-contained liquid in the petri dish.

According to another aspect of the present invention, a substance injection device includes the above petri dish to trap cells and a substance injecting unit to inject a substance into the trapped cells.

The above and other objects, features, advantages and technical and industrial significance of this invention will be better understood by reading the following detailed description of presently preferred embodiments of the invention, when considered in connection with the accompanying drawings.

## BRIEF DESCRIPTION OF THE DRAWINGS

Fig. 1 is a schematic of cell-trapping petri dish according to a first embodiment of the present invention;
Fig. 2 is a perspective for explaining the structure of a liquid retention channel;
Fig. 3 is a schematic of a cell-trapping petri dish according to a second embodiment of the present invention;
Fig. 4 is a diagram for explaining the number of parts when manufacturing the cell-trapping petri dish 60 shown in Fig. 3;
Fig. 5 is a schematic of a cell-trapping petri dish according to a third embodiment of the present invention;
Fig. 6 is a perspective of a cell-trapping petri dish according to a fourth embodiment of the present invention;
Fig. 7 is a diagram for explaining the shape of a first well shown in Fig. 6;
Fig. 8 is a schematic of a PBS supply-discharge system according to the fourth embodiment;
Fig. 9 is a diagram for explaining the preprocess for injecting a gene or a drug into a cell;
Fig. 10 is a diagram for explaining a conventional microinjection method;
Fig. 11 is a diagram for explaining the pressure state when cells are trapped using the conventional petri dish;
Fig. 12 is a diagram for explaining Laplace pressure $P_r$;
Fig. 13 is a diagram for explaining the conventional PBS supply-discharge system; and
Fig. 14 is a diagram for explaining the slope portion in a first well where a cell trapping chip is provided.

## DETAILED DESCRIPTION OF THE PREFERRED EMBODIMENTS

Exemplary embodiments of a cell-trapping petri dish according to the present invention are explained in detail below with reference to the attached drawings.

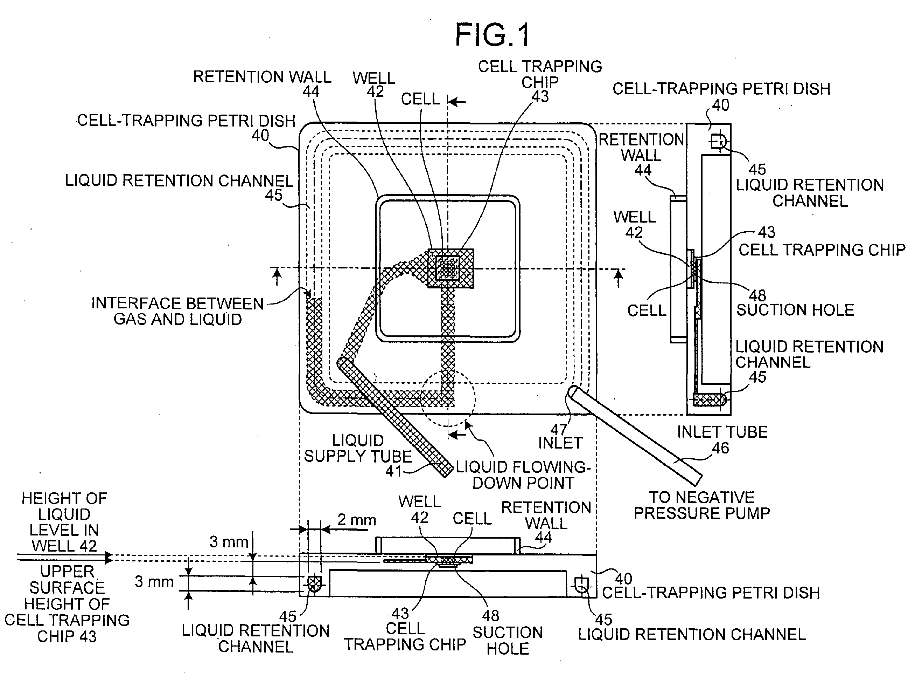

The structure of a cell-trapping petri dish according to a first embodiment of the present invention is explained first. Fig. 1 is a schematic of a cell-trapping petri dish 40 according to a first embodiment of the present invention. The cell-trapping petri dish 40 includes a well 42 for retaining PBS (Phosphate Buffer Saline) which is supplied through a liquid supply tube 41; a cell trapping chip 43 for trapping cells by sucking the PBS through suction holes 48; a retention wall 44 for retaining the PBS, which may overflow from the well 42, inside the well 42; a liquid retention channel 45 for retaining the liquid sucked through the suction holes 48 of the cell trapping chip 43; and an inlet 47 connecting between an inlet tube 46 connected to a negative pressure pump and the liquid retention channel 45.

When the liquid is sucked through the suction holes 48 formed in the cell trapping chip 43 and cells are trapped, the liquid retention channel 45 has at least a capacity for allowing the liquid sucked to be retained in the liquid retention channel 45. Further, the liquid retention channel 45 is formed so that an interface between the liquid retained in the liquid retention channel 45 and the gas therein is set in a lower position than the level of the liquid in the well 42 for soaking the cells trapped in the cell trapping chip 43.

Particularly, when the liquid retention channel 45 is formed with a hydrophobic material, the liquid retention channel 45 is formed so that a difference between the height of the interface of the liquid with the gas formed in the liquid retention channel 45 and the height of the liquid level in the well 42 cancels out the Laplace pressure produced on the interface when the liquid supply tube 41 and the inlet tube 46 are removed, and is formed so that the height is set so as to produce at least the pressure under which cells are trapped.

More specifically, the liquid retention channel 45 is arranged so that a difference between the heights is greater

than

$$P_r/\rho g$$

where $P_r$ is Laplace pressure, $\rho$ is liquid density, and g is gravity acceleration. This equation can be obtained as explained with reference to Fig. 11.

By configuring the cell-trapping petri dish 40 in the above manner and retaining the liquid sucked in the liquid retention channel 45, trapping pressure for trapping cells in the cell trapping chip 43 can be maintained even when the cell-trapping petri dish 40 is carried after the negative pressure pump is stopped and the inlet tube 46 is removed. Furthermore, because the liquid does not flow into the inlet tube 46, the liquid can be prevented from leaking to the outside.

Here, the cross-section of the liquid retention channel 45 is formed to a shape so that the influence of a capillary phenomenon is greater than the influence of the gravity. This allows prevention of the liquid from being discontinuous in the liquid retention channel 45, thus, obtaining a stable pressure state of the liquid.

More specifically, when the size of the interface between the liquid and the gas becomes larger than twice as long as the capillary length, the influence of the gravity is greater than the influence of the interface tension. Therefore, the dimension of the cross-section of the liquid retention channel 45 is set to twice or less than the capillary length, so that the influence of the interface tension becomes dominant in the liquid retention channel 45.

The capillary length mentioned here is an amount expressed by $\sqrt{\gamma/((\rho 1-\rho g)/g)}$ where $\gamma$ is interface tension, $\rho 1$ is liquid density, $\rho g$ is gas density, and g is gravity acceleration. Here, if $\rho 1 > \rho g$, the gas density is negligible, and the capillary length is expressed by $\sqrt{\gamma/\rho_1/g}$

The dimension of the cross-section indicates a longer side between the longest side in the vertical direction of the cross-section and the longest side in the horizontal direction thereof. For example, if a cross-sectional shape is a circle, the longer side is the diameter thereof, and if the cross-sectional shape is a rectangle, the longer side is a longer one of the vertical side and the horizontal side of the rectangle. If the cross-sectional shape is a shield shape as shown in Fig. 1 and the longest side in the horizontal direction of the cross-section is 2 millimeters and the longest side in the vertical direction thereof is 3 millimeters, the longest side in the vertical direction is longer than the longest side in the horizontal direction, and hence, the dimension of its cross section is 3 millimeters that is the longest side in the vertical direction.

The liquid retention channel 45 is formed so that the upper surface position of the liquid retention channel 45, in a portion where an interface between the liquid and the gas exists after completion of cell trapping, is lower by 3 millimeters than the upper surface height of the cell trapping chip 43 for trapping cells. The liquid for soaking cells trapped therein is poured up to a level higher than the upper surface height of the cell trapping chip 43. Therefore, by forming the liquid retention channel 45 in the above manner, the interface between the liquid and the gas in the liquid retention channel 45 can be surely positioned in a lower side than the level of the liquid for soaking the cells therein.

If a contact angle, which is an angle formed by the interface between the liquid and the gas in the liquid retention channel 45 and the surface of the liquid retention channel 45, is 75 degrees, the Laplace pressure is -31 Pa. Further, because the central position of the liquid retention channel 45 is lower by 4.5 millimeters than the upper surface of the cell trapping chip 43, the liquid column pressure is -44 Pa(=-4.5 millimeters x9.8 Pa/mm) assuming the pressure gradient of the liquid is 9.8 Pa/mm. Therefore, the trapping pressure for trapping cells becomes -75 Pa (=-31-44), and because this is a negative value, the trapped state of cells can be maintained even if the suction of the liquid is stopped.

The liquid retention channel 45 is formed around the cell trapping chip 43 so as to surround the cell trapping chip 43. This is because the liquid retention channel 45 is provided so as not to interfere with observation of the cells trapped in the cell trapping chip 43 by the microscope.

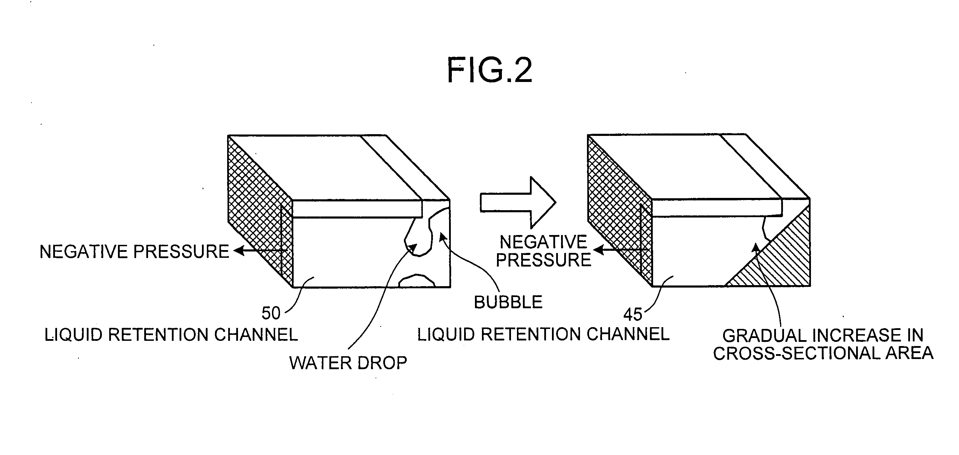

Furthermore, the liquid retention channel 45 is formed so that the height of the liquid retention channel 45 is changed and its cross-sectional area gradually increases in a location where the liquid flows down (a liquid flowing-down point in Fig. 1). Fig. 2 includes perspectives of a conventional liquid retention channel 50 and a liquid retention channel 45 according to the present embodiment. Cross-sectional area of both the liquid retention channels gradually increase.

In the conventional liquid retention channel 50, the cross-sectional area abruptly changes in a location where the liquid flows down. If the liquid retention channel 50 is formed in the above manner, water drops and bubbles are formed in the liquid retention channel 50, and the pressure state becomes unstable, which may cause the cells trapped in the cell trapping chip 43 to separate from the cell trapping chip 43.

Therefore, the liquid retention channel 45 is formed so that its cross-sectional area gradually increases, thereby minimizing water drops and bubbles to be formed, stabilizing the pressure state, and continuing the state where the cells are trapped.

As explained above, in the first embodiment, the cell-trapping petri dish 40 includes the cell trapping chip 43.

The cell trapping chip 43 includes the suction holes 48. Cells are trapped in the suction holes 48 by sucking the liquid in the cell-trapping petri dish 40 through the suction holes 48. Further, the cell-trapping petri dish 40 includes the liquid retention channel 45 formed so as to have a capacity, when the liquid is sucked through the suction holes 48 and the cells are trapped, for allowing the liquid sucked to be retained inside the liquid retention channel 45, and formed so that the interface between the liquid retained and the gas is positioned in a lower side than the level of the liquid for soaking the cells trapped in the cell trapping chip 43 therein. Therefore, even when the cell-trapping petri dish 40 is carried, the state where the cells are trapped in the suction holes 48 can be maintained, and leakage of the liquid can be prevented when the cell-trapping petri dish 40 is removed from the microinjection device.

In the first embodiment, the dimension related to the cross-section of the liquid retention channel 45 is set to twice or less than the capillary length. Therefore, the influence of the capillary phenomenon can be set greater than the influence of the gravity. This allows prevention of the liquid from being discontinuous in the liquid retention channel 45, thus, obtaining a stable pressure state of the liquid.

In the first embodiment, a difference in heights of the interface between the liquid retained in the liquid retention channel 45 and the gas therein and of the level of the liquid for soaking cells trapped in the cell trapping chip 43, is set to a difference in heights in which at least a pressure difference occurs, the pressure difference for canceling out the Laplace pressure for releasing the cells trapped in the cell trapping chip 43 therefrom. Therefore, even when the Laplace pressure, acting so as to release the cells from the cell trapping chip 43 when the cell-trapping petri dish 40 is carried, arises, the state where the cells are trapped in the suction holes 48 can be maintained, and the leakage of the liquid can be prevented when the cell-trapping petri dish 40 is removed from the microinjection device.

In the first embodiment, the liquid retention channel 45 is formed so that its cross-sectional area gradually increases in a location where the liquid flows down from the liquid retention channel 45. Therefore, formation of water drops and bubbles is minimized, the pressure state is stabilized, and the state where the cells are trapped can be continued.

In the first embodiment, the liquid retention channel 45 is arranged around the cell trapping chip 43. Therefore, the liquid retention channel 45 can be provided so as not to interfere with observation of the cells trapped in the cell trapping chip 43, by the microscope.

In the first embodiment, the liquid retention channel is formed so that the upper surface height of the liquid retention channel in a portion, where the interface exists between the liquid and the gas after trapping of the cells is completed, is in a lower side than the surface of the cell trapping chip. However, the liquid retention channel can be formed so that the upper surface height of the liquid retention channel in the portion coincides with the surface height of the cell trapping chip.

Formation of the liquid retention channel in this manner allows easy manufacture of the cell-trapping petri dish, thereby reducing the number of parts. In a second embodiment of the present invention, the following case is explained. The case is such that a liquid retention channel is formed so that an upper surface height of the liquid retention channel in a portion, where an interface exists between a liquid and gas after trapping of cells is completed, coincides with a surface height of a cell trapping chip.

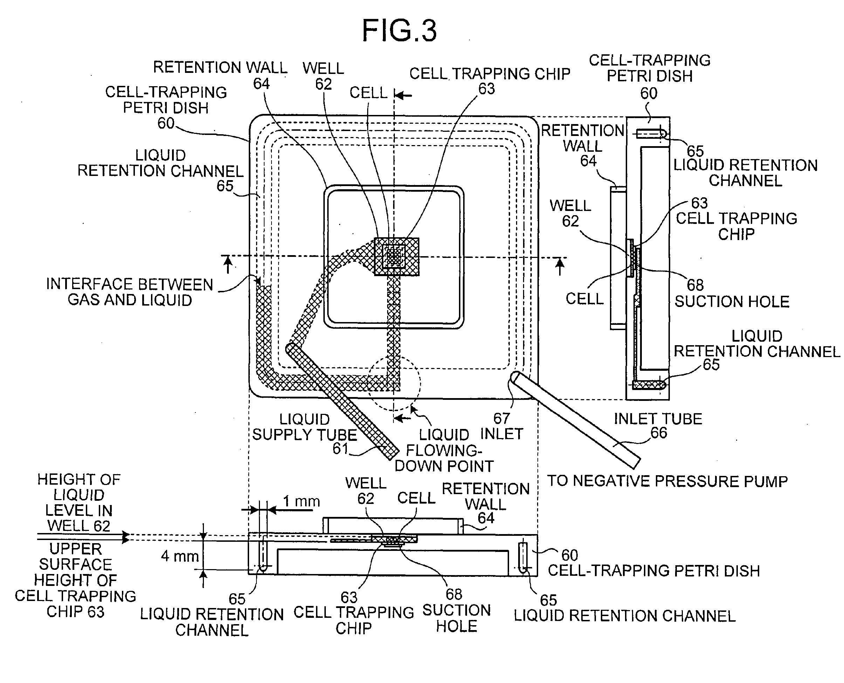

Fig. 3 is a schematic of a cell-trapping petri dish 60 according to the second embodiment. The cell-trapping petri dish 60 includes a well 62 for retaining PBS which is supplied through a liquid supply tube 61; a cell trapping chip 63 for trapping cells by sucking the PBS through suction holes 68; a retention wall 64 for retaining the PBS, which may overflow from the well 62, inside the well 62; a liquid retention channel 65 for retaining the liquid sucked through the suction holes 68 of the cell trapping chip 63; and an inlet 67 for connecting between an inlet tube 66 connected to a negative pressure pump and the liquid retention channel 65.

In the cell-trapping petri dish 60, in the same manner as the liquid retention channel 45 according to the first embodiment, the liquid retention channel 65 is formed so as to have at least a capacity, when a liquid is sucked through the suction holes 68 formed in the cell trapping chip 63 and cells are trapped, for allowing the liquid sucked to be retained in the liquid retention channel 65. Further, the liquid retention channel 65 is formed so that an interface between the liquid retained in liquid retention channel 65 and gas therein is positioned in the lower side than the level of the liquid in the well 62 for soaking the cells trapped in the cell trapping chip 63.

Furthermore, as explained with reference to Fig. 2, to suppress formation of water drops and bubbles, stabilize the pressure state, and to continue the state where cells are trapped, the liquid retention channel 65 is formed so that the height of the liquid retention channel 45 is changed and its cross-sectional area gradually increases in a location where the liquid flows down (a liquid flowing-down point in Fig. 3).

The cell-trapping petri dish 60 has a different point from the cell-trapping petri dish 40 explained in the first embodiment, in that the liquid retention channel 65 is formed so that the upper surface height of the liquid retention channel 65 in a portion, where an interface exists between the liquid and the gas after trapping of the cells is completed, coincides with the upper surface height of the cell trapping chip 63.

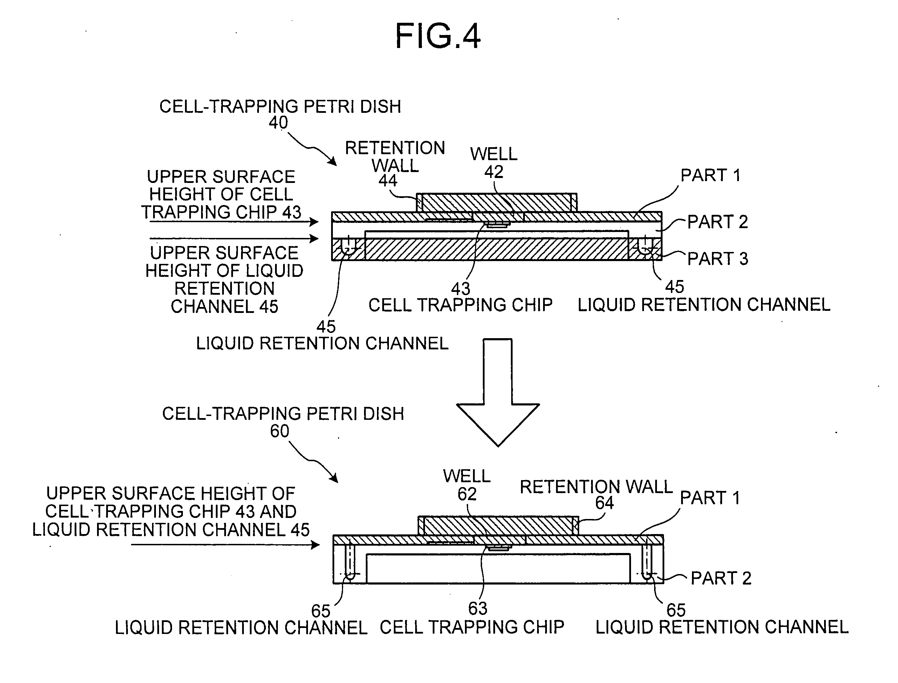

By forming the liquid retention channel 65 in the above manner, the number of parts for manufacture of the cell-trapping petri dish 60 can be reduced. Fig. 4 is a diagram for explaining the number of parts when the cell-trapping petri dish 60 is manufactured..

In Fig. 4, the number of parts of the cell-trapping petri dish 40 according to the first embodiment is compared with the number of parts of the cell-trapping petri dish 60 according to the second embodiment. In the cell-trapping petri dish 40, the upper surface height of the liquid retention channel 45 in the portion, where the interface exists between the liquid and the gas, is different from the upper surface height of the cell trapping chip 43. Therefore, it is required to produce three parts (part 1, part 2, part 3) to manufacture the cell-trapping petri dish 40 and combine the three parts.

More specifically, when the well 42, the cell trapping chip 43, and the liquid retention channel 45 are formed by machining the surface of a material, and if the upper surface height of the liquid retention channel 45 in the portion, where the interface exists between the liquid and the gas, is different from the upper surface height of the cell trapping chip 43, three parts have to be separately formed.

On the other hand, in the cell-trapping petri dish 60, the upper surface height of the liquid retention channel 65 in the portion, where the interface exists between the liquid and the gas after trapping of the cells is completed, is the same as the upper surface height of the cell trapping chip 63. Therefore, by machining one type of material, the cell trapping chip 63 and the liquid retention channel 65 can be formed, and the cell-trapping petri dish 60 can be manufactured simply by combining two parts (part 1, part 2), which allows reduction in manufacturing cost thereof.

In the example of Fig. 3, the cross-sectional shape of the liquid retention channel 65 is a shield shape with a maximum length in the horizontal direction of 1 millimeter and a maximum length in the vertical direction of 4 millimeters. In this case, if a contact angle being an angle formed by an interface between the liquid and the gas and by the surface of the liquid retention channel 65 is 75 degrees, the Laplace pressure is -46.6 Pa.

The central position of the liquid retention channel 65 is located in a lower side by 2 millimeters than a cell trapping surface of the cell trapping chip 63. Therefore, if a pressure gradient of the liquid is 9.8 Pa/mm, the liquid column pressure becomes -19.6 Pa (=-2 millimeters $\times$ 9.8 Pa/mm). Therefore, the trapping pressure for trapping cells is -66.2 Pa (=-46.6-19.6), and because this is a negative value, the state where cells are trapped can be maintained even when the suction of the liquid is stopped.

In the second embodiment, as explained above, the liquid retention channel 65 is formed so that the upper surface height of the liquid retention channel 65 coincides with the upper surface height of the cell trapping chip 63. Therefore, the number of parts upon manufacturing the cell-trapping petri dish 60 can be reduced, thereby easily manufacturing the cell-trapping petri dish 60.

In the first embodiment and the second embodiment, a material is machined to form the liquid retention channels 45 and 65, but a liquid retention channel may be formed by machining a material to form a groove and fitting a flexibly bendable tube in along the groove.

If the flexibly bendable tube is used to form the liquid retention channel, the height of the tube is changed to enable easy adjustment of the trapping pressure to an appropriate one when the trapping pressure for trapping cells is too large or too small. Therefore, in a third embodiment of the present invention, a case where a liquid retention channel is formed by forming a groove on the material and fitting a flexibly bendable tube in along the groove is explained below.

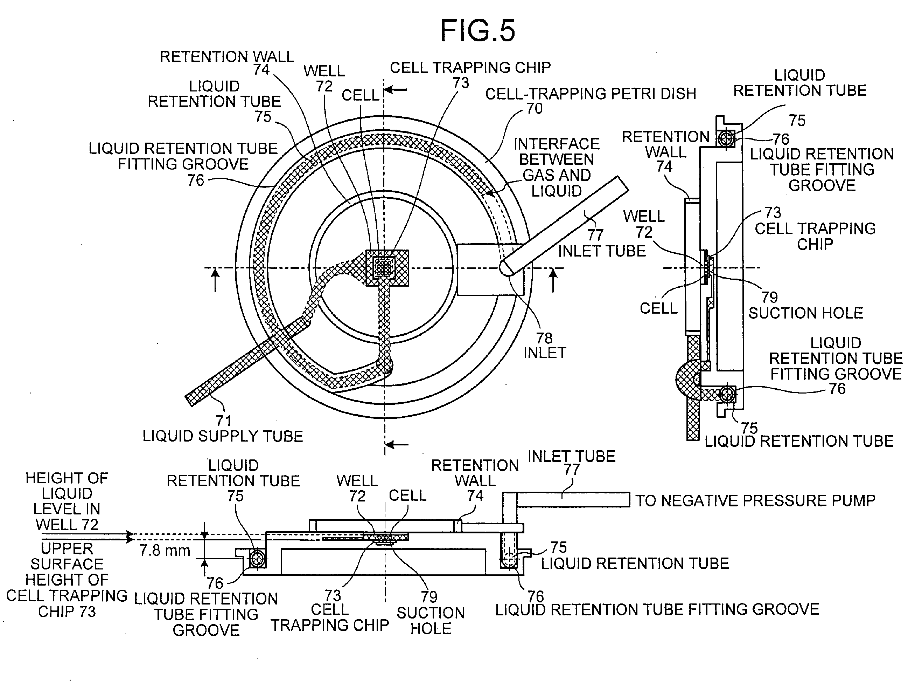

Fig. 5 is a schematic of a cell-trapping petri dish 70 according to a third embodiment. The cell-trapping petri dish 70 includes a well 72 for retaining PBS which is supplied through a liquid supply tube 71; a cell trapping chip 73 for trapping cells by sucking the PBS through suction holes 79; a retention wall 74 for retaining the PBS, which may overflow from the well 72, inside the well 72; a liquid retention tube 75 for retaining the liquid sucked through the suction holes 79 of the cell trapping chip 73; a liquid retention tube fitting groove 76 for allowing the liquid retention tube 75 to be fitted in the cell-trapping petri dish 70; and an inlet 78 connecting between an inlet tube 77 connected to a negative pressure pump and the liquid retention tube 75.

The cell-trapping petri dish 70 is different from the cell-trapping petri dish 40 or 60 according the first embodiment or the second embodiment in that the liquid retention tube fitting groove 76 for fitting the flexibly bendable liquid retention tube 75 is formed and the liquid retention tube 75 is fitted in the liquid retention tube fitting groove 76.

The liquid retention tube 75 has at least a capacity, when a liquid is sucked through the suction holes 79 formed in the cell trapping chip 73 and cells are trapped, for allowing the liquid sucked to be retained in the liquid retention tube 75. The liquid retention tube fitting groove 76 is formed so that when the liquid retention tube 75 is fitted in the liquid retention tube fitting groove 76, the interface between the liquid retained in the liquid retention tube 75 and the gas therein is positioned in a lower side than the level of the liquid in the well 72 for soaking the cells trapped in the cell trapping chip 73.

The liquid retention tube 75 can be moved vertically in the liquid retention tube fitting groove 76. Therefore, if the trapping pressure for trapping cells is too large or too small, the trapping pressure can be easily adjusted to an appropriate one by changing the height of the liquid retention tube 75.

In the example of Fig. 5, the cross-sectional shape of the liquid retention tube 75 is a circle. If the liquid retention tube 75 is hydrophobic and a contact angle, being an angle formed by an interface between the liquid and the gas and by the surface of the liquid retention tube 75, is 110 degrees, the Laplace pressure is 33 Pa.

If the central position of the liquid retention tube 75 is located in a lower side by 7.8 millimeters than the upper surface height of the cell trapping chip.73 and a pressure gradient of the liquid is 9.8 Pa/mm, the liquid column pressure becomes -76 Pa (=-7.8 millimeters ×9.8 Pa/mm). Therefore, the trapping pressure for trapping cells is -43 Pa (=33-76), and because this is a negative value, the state where cells are trapped can be maintained even when the suction of the liquid is stopped.

In the third embodiment, as explained above, the liquid sucked, when cells are trapped in the cell trapping chip 73, is retained in the flexibly bendable liquid retention tube 75. Therefore, even if the trapping pressure for trapping cells is too large or too small, the trapping pressure can be easily adjusted to an appropriate one by changing the height of the liquid retention tube 75.

In the first to the third embodiments, the cell-trapping petri dish capable of carrying while the cells are trapped in the cell trapping chip is explained, but the cell-trapping petri dish may be formed so that the surface of the cell trapping chip, where the cells are trapped, is prevented from drying when extra cells are to be removed after the cells are trapped in the cell trapping chip. Therefore, in a fourth embodiment of the present invention, a case where a cell-trapping petri dish is formed so that the surface of the cell trapping chip where the cells are trapped is prevented from drying, is explained below.

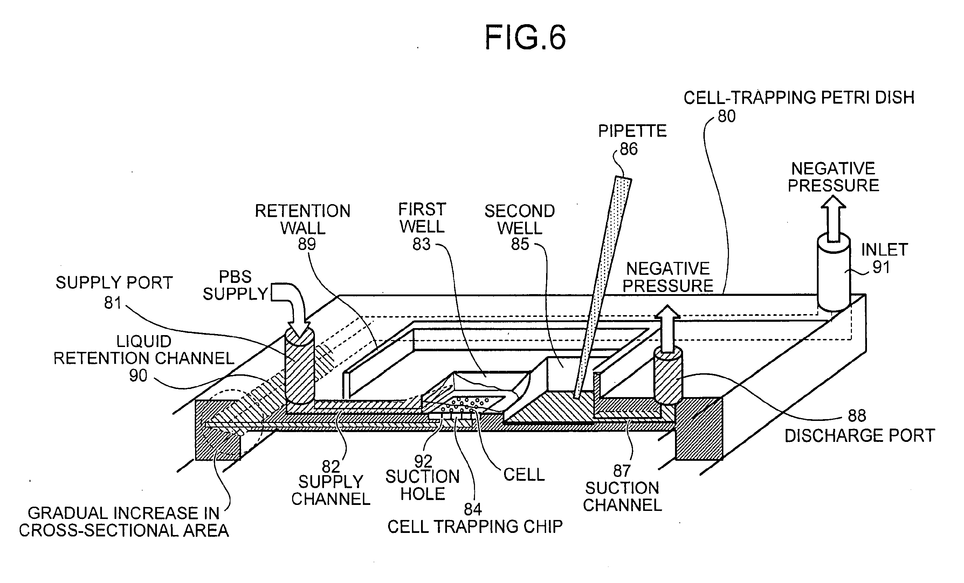

The structure of a cell-trapping petri dish according to the fourth embodiment is explained below. Fig. 6 is a perspective of a cell-trapping petri dish 80 according to the fourth embodiment.

The cell-trapping petri dish 80 includes a supply port 81 from which PBS is supplied; a supply channel 82 through which the PBS supplied through the supply port 81 is flowed into a first well 83; the first well 83 for retaining the PBS; a cell trapping chip 84 provided in the bottom of the first well 83 and for trapping cells by sucking the PBS through suction holes 92; a second well 85 for retaining PBS containing extra cells when the PBS is supplied to the first well 83 and the extra cells not trapped in the cell trapping chip 84 is flowed from the first well 83; a pipette 86 for removing the extra cells from the second well 85; a suction channel 87 for sucking the PBS containing the extra cells from the second well 85; a discharge port 88 connected with a negative pressure pump for sucking the PBS containing the extra cells through the suction channel 87; a retention wall 89 for retaining the PBS, which may overflow from the first well 83 or the second well 85, therein; a liquid retention channel 90 for retaining the PBS sucked to trap cells; and an inlet 91 connected to the negative pressure pump that sucks the PBS.

In the same manner as the cases explained in the first to the third embodiments, the liquid retention channel 90 is formed so as to have at least a capacity, when a liquid is sucked through the suction holes 92 formed in the cell trapping chip 84 and cells are trapped, for allowing the liquid sucked to be retained in the liquid retention channel 90, and formed so that the interface between the liquid retained in the liquid retention channel 90 and the gas therein is positioned in a lower side than the level of the liquid in the first well 83 for soaking the cells trapped in the cell trapping chip 84.

The liquid retention channel 90 is formed, as explained with reference to Fig. 2, so that the height of the liquid retention channel 90 is changed and its cross-sectional area gradually increases in a location where the liquid flows down, to minimize formation of water drops and bubbles, stabilize the pressure state, and to continue the state where the cells are trapped.

After the cells are trapped in the suction holes, the extra cells existing in the first well 83 are washed into the second well 85 by supplying the PBS. However, as explained with reference to Fig. 14, in the conventional petri dish, a continuous flow of the PBS produced along the wall surface of the first well 31 occurs by the influence of the surface tension, and the amount of the PBS in the first well 31 thereby decreases, which causes the surface of the cell trapping chip 30 where the cells are trapped to dry. Therefore, in the fourth embodiment, as explained in the following manner, the first well 83 is formed so as to shut off the continuous flow of the PBS.

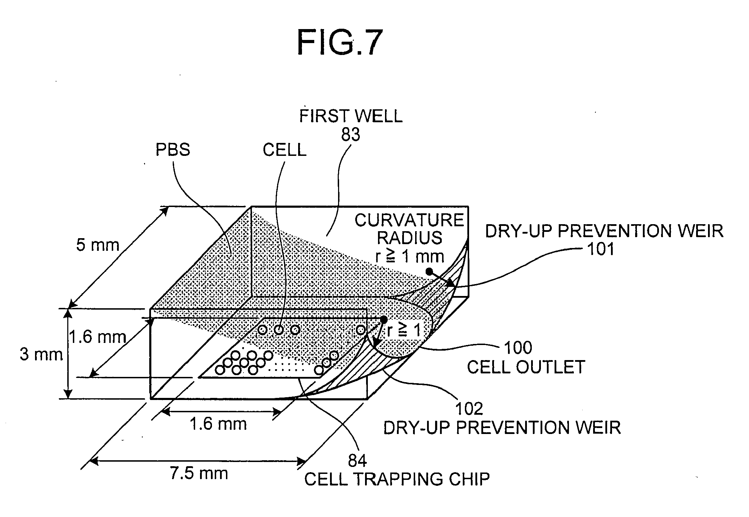

Fig. 7 is a diagram for explaining the shape of the first well 83. As shown in Fig. 7, the first well 83 has dry-up prevention weirs 101 and 102 provided at both ends of a cell outlet 100 from which the extra cells are made to flow to the second well 85.

The dry-up prevention weirs 101 and 102 are formed so as to connect between the side walls and the bottom face of the first well 83 by curved surfaces, respectively, which is different from the slope portion 33 as shown in Fig. 14. Furthermore, the height of the curved surface is getting higher toward the direction of the cell outlet 100.

It is experimentally verified that the curvature radius of the curved surface is effective if it is set to 1 millimeter or more. By setting the curvature radius of the curved surface to 1 millimeter or more, the continuous flow of the PBS produced along the wall surface of the first well 83 by the surface tension can be shut off, thereby efficiently preventing the surface of the cell trapping chip 84, where the cells are trapped, from drying.

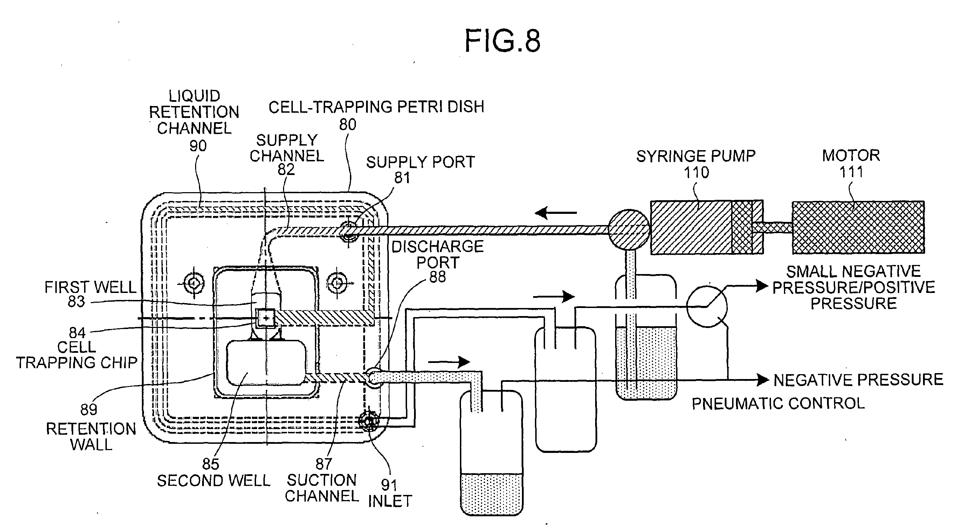

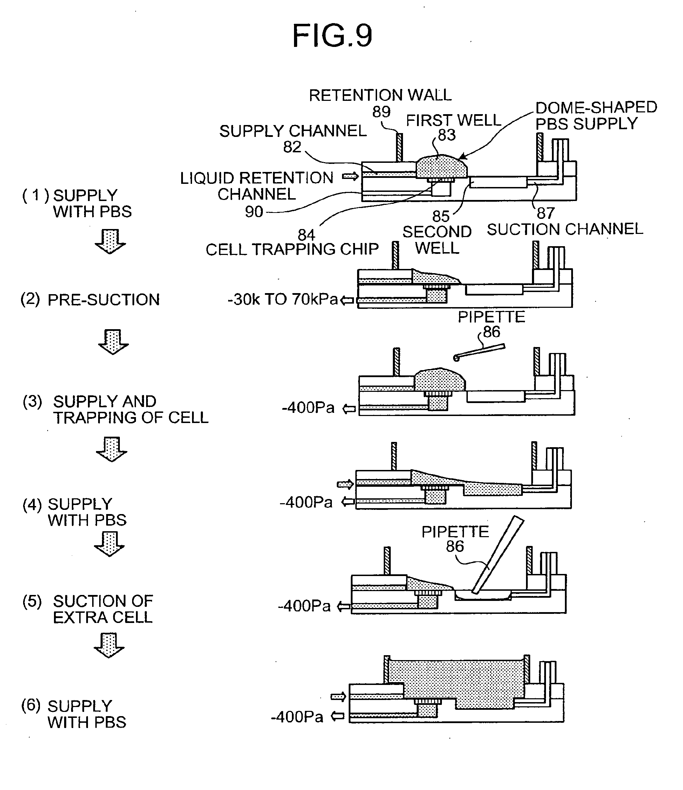

By using the cell-trapping petri dish 80 as shown in Fig. 6, there is such an advantage that there is no need to control the balance between supply and discharge of PBS unlike the conventional technology explained with reference to Fig. 13. The advantage is explained in detail below with reference to Fig. 8 and Fig. 9. Fig. 8 is a diagram for explaining a PBS supply-discharge system according to the fourth embodiment. Fig. 9 is a diagram for explaining a preprocess required for injecting a gene or a drug into a cell.

As shown in Fig. 8, the PBS supply-discharge system includes a syringe pump 110 connected to the supply port 81 of the cell-trapping petri dish 80 explained with reference to Fig. 6, and a motor 111 that drives a plunger of the syringe pump 110. Suction pumps (not shown) for performing pneumatic control are connected respectively to the discharge port 88 and the inlet 91 of the cell-trapping petri dish 80.

These components constitute the microinjection device, which injects a gene or a drug into a cell, together with the capillary needle inserted into a cell trapped in the cell trapping chip 84 to inject a gene or a drug retained inside the needle into the cell and the microscope for observing cells. The PBS supply-discharge system is used to perform the preprocess in the order as shown in Fig. 9 when a gene or a drug is injected into a cell.

As shown in Fig. 9, at first, PBS is supplied to the first well 83 by using the syringe pump 110, and the supply is continued until the PBS becomes dome-shaped by surface tension (see Fig. 9(1)). More specifically, The PBS is supplied by an amount suitable for the capacity of the first well 83. For example, if the first well 83 has dimensions as shown in Fig. 7, the supply amount of the PBS is about 113 micro litters (=5 millimeters $\times$3 millimeters $\times$7.5 millimeters).

Then, the suction pump is connected to the liquid retention channel 90, to perform pre-suction (see Fig. 9(2)). For this, a set pressure of the suction pump ranges from -30 kPa to -70 kPa. However, the set pressure P is set to the following expression so as to overcome the surface tension, which allows suction of the PBS,

$$P < -4T(\sin\theta)/d$$

where T is surface tension of water (0.072 N/m at 20 degrees centigrade), $\theta$ is a contact angle, and d is a diameter of the suction hole formed in the cell trapping chip 84.

For example, if $\theta$ is 30 degrees and d is 3 micrometers, P<-48 kPa. From this, it is understood that a suction pump should be set so that the pressure becomes a negative pressure that is smaller than about -48 kPa. It is noted that the pre-suction is performed for about 1.5 seconds.

Next, cells are supplied to the first well 83, and the process of trapping the cells in the cell trapping chip 84 is performed (see Fig. 9(3)). Here, the cell density of the PBS containing cells to be supplied is controlled so that the number of cells exists about 1.5 times as many as the number of suction holes in an area of the cell trapping chip 84 with the suction holes formed therein.

For example, if the number of suction holes in the cell trapping chip 84 as shown in Fig. 7 is 1,000 pieces, about 1,500 cells are supplied to the area. In this case, the dimensions of the area, where the suction holes are formed, in the cell trapping chip 84 is 1.6 millimeters $\times$1.6 millimeters, and the dimensions of the first well 83 is 7.5 millimeters x5 millimeters. Therefore, the number of cells to be supplied becomes as follows in terms of the whole bottom surface of the first well 83:

$$2.2 \times 10^4 \text{ pieces } (=1500 \times 7.5 \times 5/1.6/1.6)$$

If cells corresponding to this number are supplied with one drop (about 50 micro litters) of the pipette 86, the cell density of PBS containing the cells becomes about $4 \times 10^5$ pieces/ml (=$2.2 \times 10^4/50 \times 1000$).

After the cells are supplied to the first well 83, the setting of a suction pressure of the suction pump is switched to a small negative pressure, and the cells are trapped in the cell trapping chip 84. For example, if the set pressure is -400 Pa, a suction force, with which a suction hole having a diameter of 3 micrometers sucks a cell, is 2.8 nN. The cells are left as they are for about three minutes until the cells are trapped in 1000 suction holes.

Thereafter, to remove extra cells which are not trapped in the suction holes, the PBS is additionally supplied to the first well 83 and the extra cells are flowed into the second well 85 (see Fig. 9(4)). At this time, to prevent the cells trapped in the suction holes from being released therefrom, the PBS is supplied at a small flow rate. More specifically, 1 milliliter of PBS is supplied to the first well 83 at a flow rate of about 15 ml/min.

Then, the PBS containing extra cells in the second well 85 is sucked by using the pipette 86 or the suction pump connected to the suction channel 87 (see Fig. 9(5)). A flow rate for sucking the PBS containing the extra cells is set to one that does not disturb the state where the cells are trapped (e.g., 5 ml/min).

Here, a suction pressure $P_b$ required for sucking the liquid with viscosity $\eta$(Pa·s) at a predetermined flow rate Q (m3/s) by using a circular tube having an internal diameter d (m) and a length L (m) is calculated by using the following Hagen-poiseuille law.

$$P_b = -128QL\eta/\pi d^4$$

For example, if a liquid as follows is sucked, the liquid having a viscosity of $\eta=1.05\times10\text{-}3$ (Pa·s) at a flow rate of Q=5(ml/min) using a Teflon (TM) tube of d=0.76(millimeters) and L=500(millimeters), suction pressure becomes $P_b$=-5.3 kPa.

To stabilize the liquid level of the first well 83 after the PBS containing extra cells is removed from the second well 85, a predetermined amount of the PBS (e.g., 1 ml) is supplied again at a low flow rate (see Fig. 9(6)).

Thereafter, a capillary needle is inserted into the cell trapped in the cell trapping chip 84 and a gene or a drug retained in the needle is injected into the cell. The cell-trapping petri dish 80 is removed from the microinjection device and is put in an incubator (not shown) for culturing the cell.

After the gene or the drug is injected into the cell, the cell can be collected using the cell-trapping petri dish 80 explained in the fourth embodiment. More specifically, by sucking the PBS containing the extra cells from the second well 85, the second well 85 is made empty and is washed.

Then, by applying a positive pressure to the suction holes in the cell trapping chip 84 where cells are trapped, the cell injected with the gene or the drug is released from the suction hole, and at the same time, the PBS is additionally supplied to the first well 83, and the cells released.from the suction holes are flowed into the second well 85.

Thereafter, the PBS containing the cells is sucked from the second well 85 to collect the cell. In this case, the PBS is desirably sucked with the pipette 86 to reduce the damage to the cell.

In the fourth embodiment, as explained above, the cell-trapping petri dish 80 further includes the first well 83 for retaining the liquid containing cells to be trapped in the cell trapping chip, and the second well 85 for retaining the liquid flowing out of the first well 83. The first well 83 includes the dry-up prevention weirs 101 and 102 each having the curved surface which connects between the side wall and the bottom face of the first well 83 and of which height is getting higher toward the direction of the cell outlet 100 through which the liquid flows. Therefore, the liquid is flowed from the first well 83 into the second well 85 by the surface tension, thereby preventing the surface of the cell trapping chip 84 in the first well 83, where the cells are trapped, from drying.

In the fourth embodiment, the curvature radius of the curved surface is 1 millimeter or more. Therefore, the liquid is flowed from the first well 83 into the second well 85 by the surface tension, thereby more effectively preventing the surface of the cell trapping chip 84 in the first well 83, where the cells are trapped, from drying.

In the fourth embodiment, the discharge port 88 is further provided. The discharge port 88 is connected with the suction pump for discharging the liquid retained in the second well 85. Therefore, the liquid containing the cells not trapped in the cell trapping chip 84 can be effectively discharged.

In the fourth embodiment, the microinjection device includes the cell-trapping petri dish 80 and a needle for injecting a substance into a cell trapped in the cell trapping chip 84 provided in the cell-trapping petri dish 80. The needle is used to inject a substance into the cell trapped in the cell trapping chip 84 in the following case. That is, the liquid and cells are supplied to the first well 83 provided in the cell-trapping petri dish 80, to trap the cells in the cell trapping chip 84 by sucking the liquid through the suction holes in the cell trapping chip 84, and then, by supplying a liquid to the first well 83, cells not trapped in the cell trapping chip 84 are flowed into the second well 85, and the cells are removed. Therefore, there is no need to control the balance between supply and discharge of the liquid upon removal of the cells. Moreover, the substance can be injected into the cells while preventing the surface of the cell trapping chip 84, where the cells are trapped, in the first well 83 from drying caused by flowing of the liquid into the second well 85 by the surface tension.

Although the embodiments according to the present invention are explained so far, the present invention may be implemented in various embodiments, other than the embodiments explained above, within a technological scope described in claims.

Among the processes explained in the embodiments, the whole or part of the processes explained as these automatically executed can be manually executed, or the whole or part of the processes explained as these manually executed can also be automatically executed using known methods.

In addition to these, information including the process procedure, the control procedure, the specific names, and various data and parameters shown in the document and the drawings can be arbitrarily changed unless otherwise specified.

According to the present invention, the state where the cells are trapped in the suction holes can be maintained even during the carriage of the cell-trapping petri dish and the leakage of the liquid can be prevented when the cell-trapping petri dish is removed from a device.

According to the present invention, the influence of a capillary phenomenon can be set greater than the influence of the gravity, and that the liquid in the liquid retaining portion can be prevented from being discontinuous, to obtain a stable pressure state of the liquid.

According to the present invention, even if the Laplace pressure, for acting so as to release the cells from the cell trapping portion when the cell-trapping petri dish is carried, arises, the state where the cells are trapped in the suction holes can be maintained and the leakage of the liquid can be prevented when the cell-trapping petri dish is removed from the device.

According to the present invention, the number of parts used to manufacture a cell-trapping petri dish can be reduced, and accordingly, the cell-trapping petri dish can be easily manufactured.

According to the present invention, when the trapping pressure for trapping cells becomes too large or too small, the trapping pressure can be easily adjusted to an appropriate magnitude by changing the height of the tube.

According to the present invention, formation of water drops or bubbles can be suppressed and the pressure state can be stabilized to allow the state where the cells are trapped to be continued.

According to the present invention, the liquid retaining portion can be provided so as not to interfere with observation of the cells trapped in the cell trapping portion by the microscope.

According to the present invention, the liquid flows from the first retaining portion into the second retaining portion by the surface tension, and that the surface of the cell trapping portion in the first retaining portion, where the cells are trapped, can be prevented from drying.

According to the present invention, the liquid flows from the first retaining portion into the second retaining portion by the surface tension, and that the surface of the cell trapping portion in the first retaining portion, where the cells are trapped, can be more effectively prevented from drying.

According to the present invention,the liquid containing the cells not trapped in the cell trapping portion can be efficiently discharged.

According to the present invention, there is no need to control the balance between the supply and the discharge of the liquid when the cells are to be removed, and that a substance can be injected while preventing the surface of the cell trapping portion, where the cells are trapped in the first retaining portion, from drying because of flowing of the liquid into the second retaining portion by the surface tension.

Although the invention has been described with respect to a specific embodiment for a complete and clear disclosure, the appended claims are not to be thus limited but are to be construed as embodying all modifications and alternative constructions that may occur to one skilled in the art that fairly fall within the basic teaching herein set forth.

**Claims**

1. A petri dish that includes a cell trapping portion having a plurality of suction holes, a cell-contained liquid containing biological cells is placed in the petri dish and the cell-contained liquid is sucked from the suction holes, which are smaller that the cells, from below the petri dish to trap the cells in the suction holes, comprising:

   a liquid retaining portion

   having a space of a capacity that allows sucked-liquid, which is liquid sucked through the suction holes and that do not contain cells, to be retained therein, and
   configured so that an interface between the sucked-liquid in the space and gas being positioned at a lower level than a surface level of the cell-contained liquid in the petri dish.

2. The petri dish according to claim 1, wherein a cross-section of the liquid retaining portion is equal to or less than twice as long as a capillary length.

3. The petri dish according to claim 1, wherein a difference in positions of the interface and the surface level is such that at least a pressure difference occurs, the pressure difference canceling out Laplace pressure arising in the interface and releasing the cells trapped in the suction holes.

4. The petri dish according to claim 1, wherein the liquid retaining portion is formed so that an upper surface height of the liquid retaining portion coincides with an upper surface height of the cell trapping portion.

5. The petri dish according to claim 1, wherein the liquid retaining portion includes a flexibly tube.

6. The petri dish according to claim 1, wherein the liquid retaining portion is configured so that a cross-sectional area of the liquid retaining portion gradually increases in a location where the liquid flows down from the liquid retaining portion.

7. The petri dish according to claim 1, wherein the liquid retaining portion is arranged around the cell trapping portion.

8. The petri dish according to claim 1, further comprising:

a first retaining portion that retains the cell-containing liquid; and
a second retaining portion that retains the liquid flowing out of the first retaining portion, wherein
the first retaining portion includes weirs having curved surfaces each of which connects between each side face
and a bottom face of the first retaining portion, and the weirs become higher toward a direction of an outlet
where the liquid flows.

9. The petri dish according to claim 8, wherein the curvature radius of the curved surface is equal to or more than 1 millimeter.

10. The petri dish according to claim 8, further comprising an outlet connected with a discharge unit for discharging the liquid retained in the second retaining portion.

11. A substance injection device, comprising:

the cell-trapping petri dish according to claim 8; and
a substance injecting unit for injecting a substance into a cell trapped the suction holes of the cell trapping portion, wherein
when the cell-contained liquid is supplied to the first retaining portion and cells are trapped in the suction holes by sucking the cell-contained liquid from the suction holes, and when a washing liquid, which is a liquid used to wash away cells that are not trapped in the suction holes, is supplied to the first retaining portion to wash away not-trapped cells,
the substance injecting unit injects a substance into the cell trapped in the suction holes.

# FIG.1

RETENTION WALL 44
WELL 42
CELL
CELL TRAPPING CHIP 43

CELL-TRAPPING PETRI DISH 40

CELL-TRAPPING PETRI DISH 40

RETENTION WALL 44

LIQUID RETENTION CHANNEL 45

LIQUID RETENTION CHANNEL 45

WELL 42

43 CELL TRAPPING CHIP

INTERFACE BETWEEN GAS AND LIQUID

CELL

48 SUCTION HOLE

LIQUID RETENTION CHANNEL 45

LIQUID SUPPLY TUBE 41

LIQUID FLOWING-DOWN POINT

47 INLET

TO NEGATIVE PRESSURE PUMP

INLET TUBE 46

HEIGHT OF LIQUID LEVEL IN WELL 42

UPPER SURFACE HEIGHT OF CELL TRAPPING CHIP 43

WELL 42
CELL
RETENTION WALL 44

2 mm

3 mm

3 mm

40 CELL-TRAPPING PETRI DISH

45 LIQUID RETENTION CHANNEL

43 CELL TRAPPING CHIP

48 SUCTION HOLE

45 LIQUID RETENTION CHANNEL

EP 1 762 608 A1

# FIG.2

NEGATIVE PRESSURE ←

NEGATIVE PRESSURE ←

BUBBLE

50

LIQUID RETENTION CHANNEL

WATER DROP

45

LIQUID RETENTION CHANNEL

GRADUAL INCREASE IN
CROSS-SECTIONAL AREA

EP 1 762 608 A1

# FIG.3

RETENTION WALL  WELL  CELL TRAPPING CHIP  CELL-TRAPPING
64  62  CELL  63  PETRI DISH
60

CELL-TRAPPING
PETRI DISH
60

LIQUID
RETENTION CHANNEL
65

RETENTION
WALL
64

WELL
62

LIQUID RETENTION
CHANNEL

63
CELL TRAPPING
CHIP

INTERFACE BETWEEN
GAS AND LIQUID

CELL

68
SUCTION HOLE

LIQUID
RETENTION CHANNEL
65

LIQUID
SUPPLY TUBE
61

67
LIQUID INLET
FLOWING-
DOWN POINT

INLET TUBE
66

TO NEGATIVE PRESSURE PUMP

HEIGHT OF
LIQUID
LEVEL IN
WELL 62

UPPER
SURFACE
HEIGHT OF
CELL TRAPPING
CHIP 63

1 mm

WELL
62

CELL

RETENTION
WALL
64

4 mm

60
CELL-TRAPPING PETRI DISH

65
LIQUID RETENTION
CHANNEL

63
CELL TRAPPING
CHIP

68
SUCTION
HOLE

65
LIQUID RETENTION CHANNEL

EP 1 762 608 A1

FIG.4

# FIG.5

RETENTION WALL
74

WELL
72 CELL

CELL TRAPPING CHIP
73

LIQUID
RETENTION TUBE
75

LIQUID
RETENTION TUBE
75

CELL-TRAPPING PETRI DISH
70

LIQUID
RETENTION TUBE
75

LIQUID RETENTION TUBE
FITTING GROOVE
76

INTERFACE
BETWEEN
GAS AND
LIQUID

RETENTION
WALL
74

76

LIQUID RETENTION
TUBE FITTING
GROOVE

WELL
72

73
CELL TRAPPING
CHIP

77
INLET TUBE

CELL

79
SUCTION HOLE

78
INLET

LIQUID
RETENTION TUBE
FITTING GROOVE
76

71
LIQUID SUPPLY TUBE

75
LIQUID RETENTION TUBE

LIQUID
RETENTION TUBE
75

WELL
72 CELL

RETENTION
WALL
74

INLET TUBE
77

TO NEGATIVE PRESSURE PUMP

HEIGHT OF
LIQUID
LEVEL IN
WELL 72

UPPER
SURFACE
HEIGHT OF
CELL TRAPPING
CHIP 73

7.8 mm

76

LIQUID RETENTION
TUBE FITTING
GROOVE

73
CELL
TRAPPING CHIP

79
SUCTION
HOLE

75
LIQUID RETENTION TUBE

76
LIQUID RETENTION TUBE FITTING GROOVE

EP 1 762 608 A1

# FIG.6

CELL-TRAPPING PETRI DISH
80

PIPETTE
86

NEGATIVE
PRESSURE

RETENTION
WALL
89

FIRST
WELL
83

SECOND
WELL
85

INLET
91

SUPPLY PORT
81

PBS
SUPPLY

NEGATIVE
PRESSURE

LIQUID
RETENTION CHANNEL
90

88
DISCHARGE PORT

GRADUAL INCREASE IN
CROSS-SECTIONAL AREA

82
SUPPLY
CHANNEL

92
SUCTION
HOLE

84
CELL TRAPPING CHIP

CELL

87
SUCTION
CHANNEL

EP 1 762 608 A1

# FIG.7

FIRST WELL
83

PBS    CELL

CURVATURE
RADIUS
$r \geqq 1$ mm

DRY-UP PREVENTION WEIR
101

5 mm

1.6 mm

3 mm

1.6 mm

$r \geqq 1$

100
CELL OUTLET

102
DRY-UP PREVENTION WEIR

7.5 mm

84
CELL TRAPPING CHIP

EP 1 762 608 A1

# FIG.8

LIQUID
RETENTION
CHANNEL
90

CELL-TRAPPING PETRI DISH
80

SUPPLY
CHANNEL
82

SUPPLY PORT
81

SYRINGE PUMP
110

MOTOR
111

DISCHARGE
PORT
88

SMALL NEGATIVE
PRESSURE/POSITIVE
PRESSURE

FIRST WELL
83

84

CELL
TRAPPING CHIP

NEGATIVE PRESSURE

PNEUMATIC CONTROL

89
RETENTION
WALL

85
SECOND WELL

87
SUCTION
CHANNEL

91
INLET

EP 1 762 608 A1

# FIG.9

(1) SUPPLY WITH PBS

(2) PRE-SUCTION

(3) SUPPLY AND TRAPPING OF CELL

(4) SUPPLY WITH PBS

(5) SUCTION OF EXTRA CELL

(6) SUPPLY WITH PBS

RETENTION WALL
89

SUPPLY CHANNEL
82

FIRST WELL
83

DOME-SHAPED PBS SUPPLY

LIQUID RETENTION CHANNEL
90

84
CELL TRAPPING CHIP

85
SECOND WELL

87
SUCTION CHANNEL

-30k TO 70kPa

PIPETTE
86

-400Pa

-400Pa

PIPETTE
86

-400Pa

-400Pa

# FIG.10

GENE/DRUG

MICROSCOPE
5

CELL TRAPPING CHIP
2

CELL

CELL SUSPENSION

4
NEEDLE

3
SUCTION HOLE    SUCKED

1
PETRI DISH

# FIG.11

WELL
11

12
PETRI DISH

13
DRAINAGE CHANNEL

CELL

TUBE
14

CONNECTOR
15

WELL
11

CELL $P_a$ LIQUID

TO SUCTION PUMP

PETRI DISH
12

10
SUCTION HOLE $P_2=P_1-\rho gh_1$

13
DRAINAGE CHANNEL

$P_1=P_a+P_r$ $P_a$

$h_1$

EP 1 762 608 A1

# FIG.12

LAPLACE PRESSURE $P_r = -2\gamma\cos\theta/r$
$\gamma$ : SURFACE TENSION

# FIG.13

DRAINAGE CHANNEL
22

PBS SUPPLY
CHANNEL
23

PETRI DISH
20

PBS SUPPLIED/REMOVED

POSITIVE
PRESSURE

PNEUMATIC CORPORATION
CONTROL

RE-
LEASE  TRAP-
PING

SMALL NEGATIVE
PRESSURE/
POSITIVE
PRESSURE

DIS-
CHARGE

NEGATIVE
PRESSURE

24
PBS DISCHARGE
CHANNEL

21
CELL TRAPPING CHIP

EP 1 762 608 A1

# FIG.14

SECOND WELL
32

FIRST WELL
31

CONTINUOUS
FLOW OF PBS

SUCKED AND
DISCHARGED

30
CELL TRAPPING CHIP

33
SLOPE PORTION

**European Patent Office**

**EUROPEAN SEARCH REPORT**

Application Number

EP 06 25 1023

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/058847 A2 (UNIV GEORGETOWN [US]; PARANJAPE MAKARAND [US]; ESRICK MARK A [US]; CUR) 1 August 2002 (2002-08-01) * paragraphs [0033], [0034], [0041] - [0043], [0051], [0054] * * claims; figures * | 1,3-6,11 | INV. C12M3/00 B01L3/00 |
| A | US 6 383 813 B1 (BAXTER GREGORY T [US] ET AL) 7 May 2002 (2002-05-07) * column 2 * * column 3, lines 42-57 * * column 4, lines 5-10 * * column 5, lines 6-42 * * column 6, lines 44-54 * * figures * | 1-11 | |
| A | EP 1 479 759 A2 (FUJITSU LTD [JP]) 24 November 2004 (2004-11-24) * the whole document * | 1,11 | |
| A | WO 00/20554 A1 (ZENECA LTD [GB]; GARMAN ANDREW JOHN [GB]; SCANLON DAVID JOHN [GB]; DOD) 13 April 2000 (2000-04-13) * claims; figures * | 1,11 | TECHNICAL FIELDS SEARCHED (IPC) C12M B01L |
| A | WO 01/19978 A (CORNELL RES FOUNDATION INC [US]) 22 March 2001 (2001-03-22) * page 2 - page 3 * * claims * | 1,11 | |
| A | EP 0 311 059 A2 (HITACHI LTD [JP]) 12 April 1989 (1989-04-12) * column 1, lines 20-30 * * claims; figures * | 1,11 | |

The present search report has been drawn up for all claims

| Place of search | Date of completion of the search | Examiner |
|---|---|---|
| Berlin | 12 January 2007 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 06 25 1023

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2007

| Patent document cited in search report | | Publication date | Patent family member(s) | | Publication date |
|---|---|---|---|---|---|
| WO 02058847 | A2 | 01-08-2002 | NONE | | |
| US 6383813 | B1 | 07-05-2002 | NONE | | |
| EP 1479759 | A2 | 24-11-2004 | CN | 1572877 A | 02-02-2005 |
| | | | JP | 2004344036 A | 09-12-2004 |
| | | | KR | 20040100932 A | 02-12-2004 |
| | | | US | 2004235143 A1 | 25-11-2004 |
| WO 0020554 | A1 | 13-04-2000 | AT | 238412 T | 15-05-2003 |
| | | | AU | 6216199 A | 26-04-2000 |
| | | | DE | 69907249 D1 | 28-05-2003 |
| | | | DE | 69907249 T2 | 29-01-2004 |
| | | | EP | 1124939 A1 | 22-08-2001 |
| | | | JP | 2002526103 T | 20-08-2002 |
| | | | US | 6846668 B1 | 25-01-2005 |
| WO 0119978 | A | 22-03-2001 | AU | 7578900 A | 17-04-2001 |
| | | | EP | 1254215 A1 | 06-11-2002 |
| EP 0311059 | A2 | 12-04-1989 | DE | 3877682 D1 | 04-03-1993 |
| | | | DE | 3877682 T2 | 06-05-1993 |
| | | | JP | 1095768 A | 13-04-1989 |
| | | | JP | 2564322 B2 | 18-12-1996 |
| | | | US | 5154814 A | 13-10-1992 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004180555 A
- JP 2004000163 A
- JP 2005014588 A
- JP 2004284756 A
- JP 2005102094 A